# EUROPEAN PATENT SPECIFICATION

(11) **EP 1 852 126 B1**
(45) Date of publication and mention of the grant of the patent: **15.05.2013**
(21) Application number: 06022033.2
(22) Date of filing: 27.03.2002
(51) Int. Cl.: A61K 39/39, A61K 9/127, C07K 14/005, C07K 14/35, C07K 14/445, C07K 14/47, A61K 36/19, A61P 37/02, A61P 35/00

(54) **Vaccine for modulating between T1 and T2 immune responses**
Impfstoff zur Modulation zwischen T1 und T2 Immunantworten
Vaccin permettant de moduler les responses immunitaires de type T 1 et T 2

(30) Priority: 27.03.2001 US 278698 P
(43) Date of publication of application: 07.11.2007
(62) Divisional of application: 02735795.3
(73) Proprietor: Oncothyreon Inc., Seattle, WA 98121 (US)
(72) Inventor: Koganty, Rao, Edmonton Alberta T6J 3R3 (CA); Krantz, Mark J., Edmonton Alberta T6N 1H1 (CA); Longenecker, Michael B., Edmonton Alberta T6N 1H1 (CA); Budzynski, Wladyslaw A., Edmonton Alberta T6R 2P6 (CA)
(74) Representative: Leoni, Richard Pietro

(56) References cited:
- WO-A-01/70265
- WO-A-96/40236
- WO-A-97/38010
- GUAN H H ET AL: "Liposomal formulations of synthetic MUC1 peptides: Effects of encapsulation versus surface display of peptides on immune responses" BIOCONJUGATE CHEMISTRY, ACS, WASHINGTON, DC, US, vol. 9, no. 4, July 1998 (1998-07), pages 451-458, XP002186768 ISSN: 1043-1802
- NEURATH A R ET AL: "ANTIBODIES TO HEPATITIS B SURFACE ANTIGEN (HBSAG) ELICITED BY IMMUNIZATION WITH A SYNTHETIC PEPTIDE COVALENTLY LINKED TO LIPOSOMES" JOURNAL OF GENERAL VIROLOGY, READING, BERKS, GB, vol. 65, no. PART 5, May 1984 (1984-05), pages 1009-1014, XP002036804 ISSN: 0022-1317
- WASSEF N M ET AL: "LIPOSOMES AS CARRIERS FOR VACCINES" IMMUNOMETHODS, vol. 4, June 1994 (1994-06), pages 217-222, XP000601673 ISSN: 1058-6687
- MILLER M D ET AL: "VACCINATION OF RHESUS MONKEYS WITH SYNTHETIC PEPTIDE IN A FUSOGENICPROTEOLIPOSOME ELICITS SIMIAN IMMUNODEFICIENCY VIRUS-SPECIFIC CD8+ CYTOTOXIC T LYMPHOCYTES" JOURNAL OF EXPERIMENTAL MEDICINE, TOKYO, JP, vol. 176, December 1992 (1992-12), pages 1739-1744, XP001050551 ISSN: 0022-1007
- FLINN NICHOLAS ET AL: "Oral absorption studies of lipidic conjugates of thyrotropin releasing hormone (TRH)-1 and luteinizing hormone-releasing hormone (LHRH)" INTERNATIONAL JOURNAL OF PHARMACEUTICS, AMSTERDAM, NL, vol. 137, no. 1, 1996, pages 33-39, XP009093268 ISSN: 0378-5173

## Description

### FIELD OF THE INVENTION

The present invention provides liposomal vaccines capable of modulating the immune response *in vivo,* particularly the humoral and cellular immune responses.

### BACKGROUND OF THE INVENTION

To effectively combat disease, a vaccine should ideally stimulate several immunological reactions, such as the production of antibodies (humoral immunity) and the mobilization of immunological cells (cellular immunity).

A cellular immune response brings about a proliferation and stimulation of T-lymphocytes, such as cytotoxic (CTL) and delayed-type hypersensitivity (DTH) T-cells, which go on to activate macrophages and impede the propagation of pathogens. The induction of a humoral response causes the body's B-cells to produce antibodies against the offending pathogen. However, some intracellular pathogens and retroviruses survive and are extremely resistant to humoral-based immune responses and require the stimulation of cyctotoxic T-cells to destroy such biological invaders.

Synthetic peptides are often used as antigenic epitopes and can be tailor-made using standard peptide synthesis technologies so that they induce minimal side effects. However, such peptides typically invoke a relatively weak immunogenic response.

Nonetheless, immunogenicity can be boosted by attaching a lipid to the synthetic peptide. It has been shown, for example, that lipidation of synthetic peptide antigens leads to the induction of strong T-cell proliferation, CTL, and antibody responses in immunized mice, chimpanzees, or humans (BenMohamed et al., Vaccine, .18, 2843-2855 (2000); Gahery-Segard et al., J. Virology., 74, 1694-1703 (2000); Seth et al., AIDS Res. Hum. Retroviruses, 16, 337-343 (2000); Tsunoda et al., Vaccine, 17, 675-685 (1999); BenMohamed et al., Eur. J. Immunol., 27, 1242-1253 (1997); Vitiello et al., J. Clin. Invest., 95, 341-349 (1995)).

A preparation of such antigens may be delivered *in vivo* using a vaccine "carrier," but the carrier itself can become the target of the host's humoral immune response. Thus,- the host's antibodies act against the vaccine carrier and not the antigenic epitope, which can result in rapid clearance of the vaccine by anti-carrier antibodies, negating the usefulness of the actual vaccine.

The incorporation of the lipid moiety of a lipopeptide into a liposome, however, proves an extremely useful way in which to deliver an antigen *in vivo* without eliciting an immune response against the carrier. However, none of these advances assist in the modulation of one immune response to another. That is, it has not previously been shown that a liposonmally-bound lipopeptide can elicit cellular and humoral immune responses by altering the number of lipids attached to a single peptide.

The present invention, however, provides a novel way of invoking and modulating between cellular and humoral immune responses by using a single antigenic peptide and a carrier that does not stimulate humoral responses against itself.

### SUMMARY OF THE INVENTION

The invention is directed to a formulation of liposomes containing immunogenic lipopeptides. The present invention is further directed to the administration of such liposomes in formulations that are capable of both invoking and modulating an immune response in an individual.

In one embodiment, the invention provides a liposome for use as a vaccine for treating cancer, tuberculosis, malaria or hepatitis B, which comprises an immunogenic dilipopeptide wherein a lipid is attached to each of two amino acids in the dilipopeptide.

In another embodiment, the invention provides a liposomal composition for use as a vaccine for treating cancer, tuberculosis, malaria or hepatitis B, comprising at least one liposome of the invention, wherein the liposome further comprises a monolipopeptide.

Described herein is a method for producing immunogenic liposomes comprised of self assembling lipids, including lipid-derivatives of immunogenic peptides. The liposomal formulation can contain and deliver either monolipopeptides, dilipopeptides, or mixtures of each to invoke and, thereby, modulate a cellular, humoral, or cellular and humoral immune responses, respectively. The peptide is an immunogenic sequence of amino acids, representing an epitope or a similar feature that is antigenic in nature.

In an embodiment the invention provides a composition that can stimulate and modulate an immune response. Such a composition comprises a liposomal vesicle, wherein the lipid bilayer of the liposomal vesicle comprises at least one immunogenic monolipopeptide and at least one dilipopeptide, wherein the percentage of the monolipopeptide varies from more than about 0 to less than about 100% and wherein the percentage of the dilipopeptide varies from more than about 0 to less than about 100%.

Also described herein is a method of stimulating a cellular immune response comprises administering to a patient an effective amount of at least one monolipopeptide and at least one dilipopeptide, wherein the monolipopeptide and the dilipopeptide are associated with the same or different liposomal vesicle. In one instance, the percentage of the monolipopeptide administered is more than about 50%. The percentage of the monolipopeptide can also be more than about 70% or more than about 90%.

Also described is a method of stimulating a humoral immune response comprising administering to a patient an effective amount of at least one monolipopeptide and at least one dilipopeptide, wherein the monolipopeptide and the dilipopeptide are associated with the same or different liposomal vesicle. In one instance, the percentage of the monolipopeptide administered is more than about 50%. The percentage of the monolipopeptide can also be more than about 70% or more than about 90%.

The present invention also encompasses compositions wherein the peptide portion of the monolipopeptide or the dilipopeptide is derived from a protein associated with a disease selected from the group consisting of tuberculosis, hepatitis B, malaria, and cancer. In a preferred embodiment, the peptide comprises at least 5 contiguous amino acids of an immunogenic region of the protein. In a more preferred embodiment, the monolipopeptide or the dilipopeptide is designed from a MUC I protein sequence. In an even more preferred embodiment, the MUC I lipopeptide comprises the sequence GVTSAPDTRPAPGSTA. In another preferred embodiment, the monolipopeptide or the dilipopeptide is designed from a tuberculosis lipopeptide comprising the sequence DQVHFQPLPPAVVKLSDALIK. In another preferred embodiment, an antigenic MUC I peptide of the present invention can be selected from any part of the sequence SGVTSAPDTRPAPGSTAPPAHGVTSAPDTRPAPGSTAPPAHGVSSL (SEQ ID NO. 1).

In another embodiment, the monolipopeptide or the dilipopeptide is designed from a tuberculosis peptide. In a more preferred embodiment, the tuberculosis peptide has the sequence, DQVHFQPLPPAVVKLSDALIK (SEQ ID NO. 2).

In yet another embodiment, the instant invention uses a lipidated antigenic hepatitis B peptide. In a preferred embodiment, the hepatitis B peptide has the sequence, IRTPPAYRPPNAPILK (SEQ ID NO. 3). In another preferred embodiment, a malaria peptide may be modified to contain lipids. In one preferred embodiment, the mataria peptide has the sequence, VTHESYQELVKKLEALEDAVK (SEQ ID NO. 4).

Thus, one aspect of the instant invention provides a liposomal composition that comprises at least one liposome that comprises at least one monolipopeptide and at least one dilipopeptide.

In a preferred embodiment, the monolipopeptide and the dilipopeptide are selected from the group consisting of MUC I, tuberculosis, malaria, and hepatitis B peptides. In a more preferred embodiment, the monolipopeptide and the dilipopeptide peptide are any sequence, in whole or in part, selected from SEQ ID NOs.1 - 4. In another preferred embodiment, the monolipopeptide and the dilipopeptide have different amino acid sequences.

In yet another preferred embodiment, the percentage of the monolipopeptide in the liposome varies from more than 0 to less than about 100% and the percentage of the dilipopeptide varies from more than 0 to less than about 100%, based on tha totalJipopeptides incorporated-in the liposome.

In one embodiment, the percentage of the monolipopeptide and the percentage of the dilipopeptide incorporated in the liposome are each about 50%.

In another embodiment, the percentage of the monolipopeptide incorporated in the liposome is from about 50 to about 99%.

In yet one more embodiment, the percentage of the dilipopeptide incorporated in the liposome is from about 50 to about 99%.

In another preferred embodiment, the monolipopeptide and the dilipopeptide are different antigenic epitopes.

Also described herein is a liposomal formulation comprising a first liposomal composition and a second liposomal composition, wherein the first liposomal composition comprises a monolipopeptide and wherein the second liposomal composition comprises a dilipopeptide.

The liposomal formulation may contain at least one monolipopeptide and at least one dilipopeptide that are selected from the group consisting of MUC I, tuberculosis, malaria, and hepatitis B peptides. The monolipopeptide and the dilipopeptide peptide may be any sequence, in whole or in part, selected from any one of SEQ ID NOs.1 - 4. The monolipopeptide and the dilipopeptide in the liposomal formulations m a y have different amino acid sequences. The monolipopeptide and the dilipopeptide may have the same amino acid sequence.

Further described herein is a method for modulating an immune response comprising administering to a patient an effective amount of at least one monolipopeptide and at least one dilipopeptide. Methods of treating an individual to modulate the individual's immune response to an immunogenic peptide, may comprise administering to said individual an effective amount of at least one monolipopeptide and at least one dilipopeptide is provided. The monolipopeptide and the dilipopeptide are selected from the group consisting of MUC I, tuberculosis, malaria, and hepatitis B peptides. The monolipopeptide and the dilipopeptide peptide may be any sequence, in whole or in part, selected from SEQ ID NOs.1 - 4.

The monolipopeptide and the dilipopeptide may be incorporated into a liposome, wherein the percentage of the monolipopeptide varies from more than 0 to less than about 100%, and wherein the percentage of the dilipopeptide varies from more than 0 to less than about 100%, based upon the total lipopeptides incorporated in the liposome. The percentage of the monolipopeptide and the percentage of the dilipopeptide incorporated in the liposome may be each about 50%. The percentage of the monolipopeptide may be from about 50 to about 99%. In one embodiment, the percentage of the dilipopeptide is from about 50 to about 99%.

The method for modulating an immune response may result in the stimulation of a humoral response. Modulation may result in the stimulation of a cellular response. The modulation may result in an increase in the intensity of at least one of a humoral response or a cellular-response.

The method for modulating an immune response may further comprise co-administering an adjuvant with the monolipopeptide and the dilipopeptide. The adjuvant may be lipid A.

Described herein is a method of modulating between a cellular and humoral immune response, comprising first administering a formulation comprising a liposomally-bound monolipopeptide and then subsequently administering a formulation comprising a liposomally-bound dilipopeptide. Alternatively, another instance, involves first administering a formulation comprising a liposomally-bound dilipopeptide and then subsequently administering a formulation comprising a liposomally-bound monolipopeptide.

The monolipopeptide and the dilipopeptide may be selected from the group consisting of MUC I, tuberculosis, malaria, and hepatitis B peptides. The monolipopeptide and the dilipopeptide peptide may be any sequence, in whole or in part, selected from SEQ ID Nos. 1 - 4.

Also described herein is a method for modulating an immune response comprising administering to an individual at least one liposome which consists essentially of a dilipopeptide. Another instance involves a method for modulating an immune response comprising administering to an individual at least one liposome which consists of a dilipopeptide. The method for modulating an immune response may comprise administering to an individual at least one liposome which comprises a dilipopeptide. The methods for modulating an immune response may further comprise co-administering an adjuvant. The adjuvant may be lipid A.

The dilipopeptide may be selected from the group consisting of MUC I, tuberculosis, malaria, and hepatitis B peptides. The dilipopeptide peptide may be any sequence, in whole or in part, selected from SEQ ID Nos. 1 - 4.

The instant invention also provides lipidated antigenic peptides. In one embodiment, the instant invention provides a MUC I peptide composition comprising one lipid attached to an amino acid of the MUC I peptide. In a preferred embodiment, the MUC I peptide comprises, in whole or in part, the sequence of SEQ ID NO. 1. In yet another preferred embodiment, the MUC I peptide comprises the sequence SGVTSAPDTRPAPGSTA or STAPPAHGVTSAPDTRPAPGSTAPP.

In another embodiment, the MUC I peptide composition further comprises a second lipid that is-also attached to an amino acid of the MUC I peptide. In one embodiment, each lipid is attached to different amino acids. In another embodiment, the lipids are positioned at either the N-terminus or the C-terminus of the MUC I peptide.

The instant invention also provides a tuberculosis peptide composition comprising one lipid attached to an amino acid of the tuberculosis peptide. In a preferred embodiment, the tuberculosis peptide comprises the sequence DQVHFQPLPPAVVKLSDALIK. In another embodiment, the tuberculosis peptide composition comprise two lipids attached to-at least one amino acid of the tuberculosis peptide. In another embodiment, each lipid is attached to different amino acids. In another embodiment, the lipids are positioned at either the N-terminus or the C-terminus of the tuberculosis peptide.

The instant invention also provides a hepatitis B peptide composition comprising one lipid attached to an amino acid of the hepatitis B peptide. In a preferred embodiment, the hepatitis B peptide comprises the sequence IRTPPAYRPPNAPILK. In another embodiment, the hepatitis B peptide composition comprises two lipids attached to at least one amino acid of the hepatitis B peptide. In another embodiment, each lipid is attached to different amino acids. In another embodiment, the lipids are positioned at either the N-terminus or the C-terminus of the hepatitis B peptide.

The instant invention also provides a malaria peptide composition comprising one lipid attached to an amino acid of the malaria peptide. In a preferred embodiment, the malaria peptide comprises the sequence VTHESYQELVKKLEALEDAVK. In another embodiment, the malaria peptide composition comprises two lipids attached to at least one amino acid of the malaria peptide. In another embodiment, each lipid is attached to different amino acids. In another embodiment, the lipids are positioned at either the N-terminus or the C-terminus of the malaria peptide.

The instant invention also provides a peptide selected from the group consisting of SEQ ID NOs 1-4, comprising more than two lipids.

In a preferred embodiment, a lipid may be selected from the group consisting of myristyl, palmitoyl and lauryl.

Also described herein is the use of a composition comprising at least one liposome into which is incorporated one or both of a monolipopeptide or a dilipopeptide of an antigenic peptide, as a vaccine tor modulating the immune response to said peptide, wherein the relative amounts of monolipopeptide and dilipopeptide modulate the relative intensities of T-cell proliferation and antibody production.

In another embodiment the invention provides a liposomal vaccine for use in stimulating an immune response in an individual which comprises an immunogenic dilipopeptide, said dilipopeptide comprising at least one peptide epitope, wherein a lipid is attached to each of two amino acids in the dilipopeptide.

Both the foregoing general description and the following brief description of the drawing and the detailed description are exemplary and explanatory and are intended to provide further explanation of the invention as claimed. Other objects, advantages, and novel features will be readily apparent to those skilled in the art from the following detailed description of the invention.

### BRIEF DESCRIPTION OF THE DRAWING

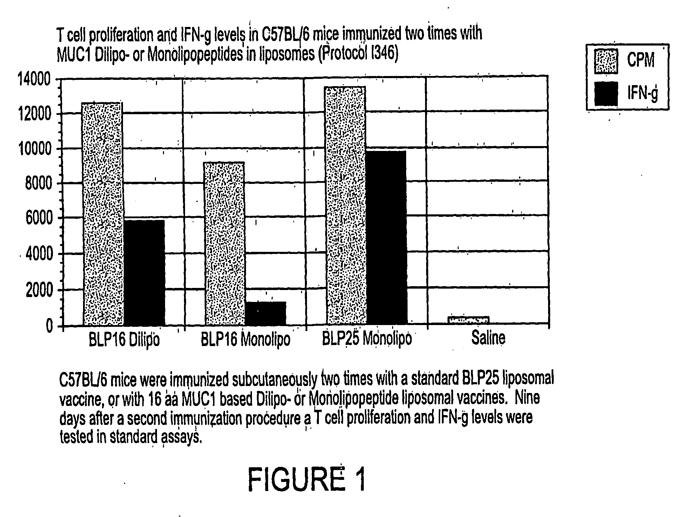

Figure 1 shows levels of T cell proliferation and IFN-γ in C57B1/6 mice immunized 2 times with MUC1 dilipo- or monolipopeptides in liposomes.

### DETAILED DESCRIPTION OF THE PREFERRED EMBODIMENTS

An unexpected and surprising finding of the present invention is that the induction of immune responses *in vivo* can be modulated by administering serially, or in combination, liposomally-bound peptides comprising one lipid chain and liposomally-bound peptides comprising at least two lipid chains.

The term "modulate" means some change, adjustment, or adaptation to a certain proportion. Thus, an immune response may be modulated by stimulating factors which bring into play a change in immunological activity. The intensity of an immune response may also be modulated. For example, the intensity or level of T-cell proliferation can be made greater or lower after administration of factors that effect modulation. In the present invention, those factors can be liposomally bound lipopeptides.

It was surprisingly discovered that a peptide having one lipid chain-may invoke a large cellular immune response (*i.e.,* a "T1" immune response) when incorporated into a liposomal formulation, but only a minimal or no humoral response. However, that same peptide, when incorporated into a liposome, can be made to induce a large humoral response (*i.e.,* a "T2" response), accompanied by either a minimal or massive cellular response, when two lipids are attached to its surface. The range of cellular response observed with a lipopeptide varies depending upon the identity of the lipopeptide. The presence of one, two, or more than two lipids on an antigenic peptide may also increase or reduce the intensity by which an immune response is activated. The intensity of an immune response and the type of immune response that is stimulated can also be modulated by varying the number of amino acid residues between two lipids on an antigenic peptide.

The present invention demonstrates that attaching more than one lipid chain to a peptide bound to a liposomal bilayer stimulates a largely humoral immune response, along with variable cellular activity ranging from minimal to massive. The present invention also shows that attaching a second lipid to an antigenic peptide increases the level of T-cell proliferation as well as inducing antibody production as compared to the monolipid derivative. The term "derivative" means a compound derived or obtained from another that contains essential elements of the parent substance. Thus, the lipid derivative of an antigen refers to a peptide that has at least one lipid attached. Hence it is possible to administer an effective amount of a liposomal formulation comprising only dilipopeptides to invoke antibody production and cellular activity. An "effective amount" of liposomally bound lipopeptide formulation, refers to an empirically-derived amount of that lipopeptide that modulates an -immune response.

It was also discovered that for some antigens a dilipopeptide lipsomal formulation will trigger a massive cellular response, in addition to a large humoral response.

Specifically, the examples show that liposomal formulations having a monolipopeptide induced largely cellular responses and minimal humoral responses, and liposomal formulations having a dilipopeptide induced largely humoral responses and minimal cellular responses, for MUC-1, tuberculosis, and hepatitis B peptides. Administering a combination of a di- and a monolipopeptide resulted in superior cellular and humoral responses.

Another advantage of the present invention is that different lipopeptides can be incorporated into a liposome and thus be transported and presented to the immune system simultaneously and under-the same conditions. Thus, it is possible to induce in an individual multiple immune responses by treating that individual with a liposome that contains a T1-inducing monolipopeptide and a T2-inducing dilipopeptide. Alternatively, a mixture of liposomally-bound monolipopeptides and liposomal-bound dilipopeptides can also be used to simultaneously induce multiple immune responses.

Thus, the present invention provides novel compositions and methods for modulating an immune response by varying the number of lipids that are attached to an antigenic peptide. An immune response can be modulated by adding a two or more lipids to an antigenic peptide, as can the intensity of the immune-response. Furthermore, the intensity of an immune response can be modulated by varying the spacing of amino acids between lipids.

An immune response can also be invoked by injecting pre-stimulated antigen-presenting cells or T cells into a patient. Known as "adoptive immunotherapy," this technique creates *in vitro* an expanded population of antigen-specific cells that are primed to combat the causative agent once reintroduced into the body. In essence, cells are removed from the patient, stimulated *in vitro* and reinjected back into the patient's bloodstream. Specifically, peripheral blood lymphocytes, such as antigen presenting cells, are isolated and then "charged" by exposing the cells to antigens *in vitro.* The antigen becomes endocytosed by an antigen presenting cell, whereupon it becomes associated with a major histocompatibility complex and subsequently presented on the outer surface of the cell. This population of primed cells can then be reinjected into the patient. Fractionating the peripheral blood lymphocytes into dendritic cells and/or macrophages prior to charging can also be performed.

Thus, a liposomal formulation of the present invention, comprising membrane-bound, antigenic lipopeptides can be added to the isolated antigen presenting cells *in vitro* to "charge" them. For example, a liposomal formulation comprising a monolipo-MUC I peptide, a dilipo-MUC I peptide, or a combination of both peptides, can be used to charge peripheral blood lymphocytes which are then reinjected into the patient as a cellular vaccine.

Alternatively, "adoptive T-cell transfer therapy" can be performed. This entails incubating a patient's T-cells with pre-charged antigen presenting cells *in vitro.* The T--cells become activated and are re-administered to a patient suffering from, for example, an adenocarcinoma. For a description of art-recognized techniques for adoptive T-cell transfer therapy, see Bartels et al., Annals of Surgical Oncology, 3(1):67 (1996). Thus, according to the present invention, a lipidated antigenic peptide is selected, incorporated into a liposome, and used to stimulate peripheral blood lymphocytes; which can either be injected back into the patient or used themselves to activate isolated T-cells. A T-cell activation method is also useful for generating cytotoxic and helper T-cell responses to antigens involved in various pathological conditions, such as cancer, tumors, viral infections, and bacterial infections.

### Lipsomes

Liposomes are microscopic vesicles that consist of one or more lipid bilayers surrounding aqueous compartments. *See e.g.,* Bakker-Woudenberg et al., Eur. J. Clin. Microbiol. Infect. Dis. 12 (Suppl. 1): S61 (1993) and Kim, Drugs, 46: 618 (1993). Because liposomes can be formulated with bulk lipid molecules that are also found in natural cellular membranes, liposomes generally can be administered safely and are biodegradable.

Depending on the method of preparation, liposomes may be unilamellar or multilamellar, and can vary in size with diameters ranging -from about 0.02 µm to greater than about 1.0 µm. A variety of agents can be encapsulated in liposomes. Hydrophobic agents partition in the bilayers and hydrophilic agents partition within the inner aqueous space(s). *See e.g.,* Machy et al., LIPOSOMES IN CELL BIOLOGY AND PHARMACOLOGY (John Libbey, 1987), and Ostro *et al., American* J. Hosp. Pharm. 46: 1576 (1989).

Liposomes can adsorb to virtually any type of cell and then release an incorporated agent. Alternatively, the liposome can fuse with the target cell, whereby the contents of the liposome empty into the target cell. Alternatively, a liposome may be endocytosed by cells that are phagocytic. Endocytosis is followed by intralysosomal degradation of liposomal lipids and release of the encapsulated agents. Scherphof et al., Ann. N.Y. Acad. Sci., 446: 368 (1985).

Other suitable liposomes that are used in the methods of the invention include multilamellar vesicles (MLV), oligolamellar vesicles (OLV), unilamellar vesicles (UV), small unilamellar vesicles (SUV), medium-sized unilamellar vesicles (MUV), large unilamellar vesicles (LUV), giant unilamellar vesicles (GUV), multivesicular vesicles (MVV), single or oligolamellar vesicles made by reverse-phase evaporation method (REV), multilamellar vesicles made by the reverse-phase evaporation method (MLV-REV), stable plurilamellar vesicles (SPLV), frozen and thawed MLV (FATMLV), vesicles prepared by extrusion methods (VET), vesicles prepared by French press (FPV), vesicles prepared by fusion (FUV), dehydration-rehydration vesicles (DRV), and bubblesomes (BSV). The skilled artisan will recognize that the techniques for preparing these liposomes are well known in the art. See COLLOIDAL DRUG DELIVERY SYSTEMS, vol. 66 (J. Kreuter, ed., Marcel Dekker, Inc., 1994).

### Lipids

A "lipid" may-be a myristyl, palmitoyl, or a lauryl molecule, that can be attached to amino-acids that possess functional oxygen, nitrogen, or sulfur groups. Such amino acids include, but are not limited to, threonine, serine, lysine, arginine, and cysteine amino acids. A "monolipopeptide" is a peptide to which only one lipid chain is attached. Similarly, a "dilipopeptide" is a peptide that has two lipid chains attached to one or two amino acids. If the two lipid chains are attached to two amino acid residues, those residues can be spaced any number of amino acids apart.

A "liposomal formulation" describes *in vitro*-created lipid vesicles in which mono- and/or dilipopeptides can be incorporated. Thus, "lipesomally-bound" refers to-a peptide that is partially incorporated or attached to a liposome. A liposomal formulation may also be referred to as a "liposomal vaccine." A liposomal formulation may comprise two types of liposomes; one that contains mostly, if not all, monolipopeptides incorporated into its structure, and a second that contains mostly, if not all, dilipopeptides in its structure. Individual preparations of "mono-" and "di-" liposomes can be administered together to modulate an immune response, even though the monolipopeptide and dilipopeptide do not exist on one liposome.

When incorporated into a liposome, the monolipopeptide and the dilipopeptide may be peptides that are the same antigenic epitope. Alternatively, the peptide sequences for each lipopeptide may comprise different epitopes. The lipopeptides may be.antigens that are associated with the same or different proteins.

A lipopeptide can be incorporated into liposomes because the lipid portion of the peptidic molecule will spontaneously integrate into the lipid bilayer. Thus, a lipopeptide may be presented on the "surface" of a liposome. Alternatively, a peptide may be encapsulated within a liposome. Techniques for preparing liposomes and formulating them with molecules such as peptides are well known to the skilled artisan.

### Exemplary Adjuvants

The present liposomal vaccines may also be formulated advantageously with an adjuvant. As conventionally known in the art, adjuvants are substances that act in conjunction with specific antigenic stimuli to enhance the specific response to the antigen. Monophosphoryl lipid A (MPLA), for example, is an effective adjuvant that causes increased presentation of liposomal antigen to specific T Lymphocytes. Alving, C.R., Immunobiol., 187:430-446 (1993). The skilled artisan will recognize that lipid-based-adjuvants, such as Lipid A and derivatives thereof, are also suitable. A muramyl dipeptide (MDP), when incorporated into liposomes, has also been shown to increase adjuvancity (Gupta RK et al., Adjuvants-A balance between toxicity and adjuvancity," Vaccine, 11, 293-306 (1993)).

Another class of adjuvants includes stimulatory cytokines, such as IL-2. Thus, the present liposomal vaccines may be formulated with IL-2, or IL-2 may be administered separately for optimal antigenic response. IL-2 is beneficially formulated with liposomes.

### Exemplary Vaccine Formulations

Vaccines may also be formulated with a pharmaceutically acceptable'excipient. Such excipients are well known in the art, but typically should be physiologically tolerable and inert or enhancing with respect to the-vaccine properties of the inventive compositions. Examples include liquid vehicles such as sterile, physiological saline. An excipient may be added at any point in formulating a liposomal vaccine or it may be admixed with the completed vaccine composition.

Vaccines may be formulated for multiple routes of administration. Specifically preferred routes include intramuscular, subcutaneous, or intradermal injection, aerosol, or oral administration, or by a combination of these routes, administered at one time or in a plurality of unit dosages. Administration of vaccines is well known and ultimately will depend upon the particular formulation and the judgement of the attending physician. Vaccine formulations can be maintained as a suspension or they may be lyophilized and hydrated later to generate a useable vaccine.

To provide greater specificity, thus reducing the risk of toxic or other unwanted effects during *in vivo* administration, it is advantageous to target the inventive compositions to the cells through which they are designed to act, namely antigen-presenting cells. This may conveniently be accomplished using conventional targeting technology to direct a liposome containing an immunogenic peptide to a particular location within the body. To target antigen presenting cells, for example, mannose and the Fc portion of antibodies can be chemically conjugated to an antigenic peptide, or by recombinantly fusing the targeting peptide to the immunogenic lipopeptide. Other, similar strategies will be familiar to the practitioner.

### Exemplary Quantities of Lipopeptides and Liposomal Formulations

The ratio of antigenic monolipopeptides and dilipopeptides within a liposome can be varied so as to modulate an immune response to different degrees of intensity. For example, increasing the amount of dilipopeptide incorporated in a liposome relative to the amount of monolipopeptide may make the resulting formulation more humoral-inducing. Of course, due to differing magnitudes of response to different antigens, different ratios may be needed to achieve the desired balance of humoral and cellular response.

For example, the skilled artisan can create liposomes made up of a ratio of covalently linked, immunogenic monolipopeptides and dilipopeptides, wherein the percentage of the monolipopeptide varies from more than about 0 to less than about 100% of the liposome. Similarly, the dilipopeptide can be present in the liposomal membrane as a percentage of more than about 0 to less than about 100%. For example, a liposome comprising 75% monolipopeptide and 25% dilipopeptide may generate a largely T1 immune response with some T2 activity. The present disclosure describes methods for creating a liposome comprising about 1 to about 30% monolipopeptide, about 30 to about 50% monolipopeptide, or about 50 to about 99% monolipopeptide. Similarly, a liposome comprising about 1 to about 30% dilipopeptide, about 30 to about 50% dilipopeptide, or about 50 to about 99% dilipopeptide can also be created. Thus, the present invention enables the creation of liposomes containing, for example, monolipopeptide: dilipopeptide in ratios such as about 10%:about 90%, about 30%:about 70%, about 50%:about 50%, about 70%:about 30%, about 90%:about 10% and about 99%:about 1%. Determining relative antigenicity is within the purview of one having ordinary skill in immunology.

An effective amount of a liposomal formulation containing at least one monolipopeptide and at least one dilipopeptide can be administered to a patient, wherein the monolipopeptide and dilipopeptide are associated with either the same or different liposomal vesicle. Thus a single liposomal formulation comprising a ratio or ratios of a monolipopeptide and a dilipopeptide can be administered to a patient to invoke a desired immune response; or alternatively, combinations of at least two liposomal formulation comprising different ratios of a monolipopeptide and a dilipopeptide can be administered to a patient to invoke a similar or different immune response.

"Treating" in its various grammatical forms refers to preventing, curing, reversing, attenuating, alleviating, minimizing, suppressing, or halting the deleterious effects of a disease state, disease progression, disease causative agent, or other abnormal condition.

### Exemplary Immunogenic Peptides Useful in the Invention

Any peptidic antigen or epitope may be lipidated and incorporated into a liposome for the purposes of inducing or modulating immune responses *in vivo.* One, two, or more than two lipids can be added to any part of a peptide. The skilled artisan will recognize that the antigenic peptide is selected based upon the type of disease affecting the individual. For example, a "MUC-1" antigen is useful for making antigen-specific T-cells that can be used in treating adenocarcinoma. Similarly, a tuberculosis peptide, hepatitis B peptide, or a malaria peptide all can be lipidated according to the present invention and used to invoke cellular and humoral immunity as desired to treat the specific diseases with which the peptides are associated. The present invention is not limited to the use of MUC-1, tuberculosis peptides, hepatitis B peptides, or malaria peptides as liposomally-bound lipopeptidic vaccines.

A variety of immunogenic peptides can be lipidated and incorporated into liposomal membranes to create a number of different immunogenic-specific vaccines. Described herein, for example, is the immunogenic effect of lipidation upon a mucin-derived peptide. MUC I mucins are macromolecular glycoproteins expressed in all epithelial cells of healthy individuals. The core peptidic sequence of MUC I comprises 20 amino acid residues that are repeated throughout the protein anywhere from 60 to 120 times. The repeated sequence, GVTSAPDTRPAPGSTAPPAH, has five potential sites for glycosylation (bolded **S**, serine and **T**, threonine) and an immunogenic "DTR" epitope (underlined).

Generally, carbohydrates are linked to one or more of the serine or threonine residues as O-linked structures and when all five sites are glycosylated, the epitope is concealed. In cancer cells, however, the glycosylation step is prematurely terminated such that the resultant carbohydrates are truncated. Consequently, the DTR epitope is exposed and the peptidic core sequence and the carbohydrates become immunogenic. Thus, this peptide sequence is one example of an epitope that may be used in the context-of the present invention so as to induce and modulate an immune response. For example, an antigenic MUC I peptide of the present invention can be selected from any part of the sequence SGVTSAPDTRPAPGSTAPPAHGVTSAPDTRPAPGSTAPPAHGVSSL (SEQ ID NO. 1) and lipidated so as to contain one, two, or more than two lipids.

The size of an immunogenic peptide is also subject to variation. Lipidated MUC-I peptidic molecules ranging from 16 (1882 Daltons) to 40 (5050 Daltons) amino acids in size, for example, invoke immune responses as described in the present invention. However, such immunogenic peptides are not limited by size and may be a portion or even all of the desired immunogen. Typically, small peptide antigens are preferred, due to ease of manufacture and greater specificity. Thus, SGVTSAPDTRPAPGSTA and STAPPAHGVTSAPDTRPAPGSTAPP are smaller MUC I peptides that can be lipidated according to the present invention. Accordingly, unless they are multimeric (*i.e.,* multiple copies of the same epitope), most antigens will comprise from about 9 to about 100 aminoacids. More-specifically, antigens that are about 9 to about 20, about 20 to about 40, about 40 to about 60, about 60 to about 80, and about 80 to about 100 amino acids in size can be lipidated and incorporated into liposomes as described. Fragments of protein antigens can be produced by recombinant DNA techniques and assayed to identify particular epitopes. Areferably, small peptides are produced by *in vitro* synthetic methods-and assayed. Thus, any antigenic sequence in whole, or in part, may be used in a lipidated form according to the instant invention. By "whole, or in part" it is meant that either the entire antigenic peptide or some smaller-peptide derived from the larger may be lipidated. A "smaller peptide" may be a fragment of a larger peptide antigen or may be synthesized recombinantly or chemically.

Other illustrative examples of peptides that can be synthesized, lipidated, and used in liposomally prepared vaccines include, but are not limited to, peptides involved in tuberculosis, hepatitis B, malaria, and cancer diseases.

The tuberculosis peptide DQVHFOPLPPAVVKLSDALIK (SEQ ID NO. 2), which originates from a 38kDa secretory protein of *Mycobacterium tuberculosis,* can be made with one or two lipids and formulated into liposomal formulations described above.

Similarly, a hepatitis B antigenic peptide is represented by the amino acid sequence IRTPPAYRPPNAPILK (SEQ ID NO. 3). Likewise, the malaria peptide VTHESYQELVKKLEALEDAVK (SEQ ID NO. 4) also can be formulated into a liposomal vaccine.

An amino acid, such as threonine, serine, lysine, arginine, or cysteine, which occurs within the natural sequence of an antigenic peptide, may be a convenient site to which a lipid can be linked. Alternatively, any one of these amino acids can be added to either end or within a peptide sequence so as to facilitate the linking of a lipid moiety. Thus, an antigenic peptide can be made to have two lysine residues at its carboxyl terminus so as to facilitate the linking of two lipids. By "made", the present invention contemplates the use of conventional peptide synthesis methods to introduce one or more additional amino acids to a peptide sequence. However, recombinant methods also can be employed to design polynucleotides that encode the desired amino acid sequence. Thus, the present invention envisions the chemical and recombinant synthesis of antigenic peptides that are amenable to lipidation.

The examples below are intended to illustrate but not limit the invention. While they are typical of those that might be used, other procedures known to those skilled in the art may be used.

### EXAMPLE 1

The purpose of this example was to determine the T-cell proliferation response and the Anti-MUC-1 antibody levels in response to administration of a MUC-1 antigen liposomal vaccine.

### SUMMARY OF PROCEDURES USED

### (i) Immunization

MUC1 based liposomal vaccines ("BLP25 liposomal vaccines") at a dose of 100 µg (250 µl) was injected subcutaneously, two, three, or four times, at biweekly intervals, into right and left inguinal regions (125 µl per each site).

### (ii) T-cell proliferation assay

Nine days after the last immunization with BLP25 liposomal vaccine all mice were sacrificed and lymph nodes were surgically excised. Nylon wool purified lymph node T cells were then cultured with antigen presenting cells (APCs), which were obtained from the spleens of naive mice of the same strain and treated with mitomycin C. These mixed cultures were pulsed with MUC1 derived synthetic lipopeptide (BP1-148) and control peptide (BP1-72) for four days. After the fourth day, some supernatants were collected for IFN-g assay, and the cultures were then pulsed with a fresh medium that contained a tritium labelled thymidine. After a further 18-20 hours the-incorporation of DNA-incorporated tritium was counted in a liquid scintillation-counter.

### (iii) IFN-gamma assay

IFN-γ levels in collected supernatants were determined by a specific ELISA using a sandwich technique. Briefly, 96 well Maxisorp flat bottom plates (Nunc, Denmark) were coated with 50 ul of catcher monoclonal antibody R4.6A2 (Biomira, lot# IM98A20A) for 35 min at 37°C, 5% CO₂. The plates were then washed and incubated 45 minutes with test samples and with positive standard cytokine sample (Pharmingen lot# M031554).

After two washes the second biotinylated antibody was added: XMG1.2 (Biomira lot# BG98G02B). After washing, peroxidase-conjugated streptavidin (Jackson ImmunoResearch lot# 42350) was added and again incubation was 30 minutes. After 5 washes, 100 µl of HRPO substrate solution: 1 µl of 30% H₂O₂ diluted in 10 mL of 1 mg/mL ABTS (Aldrich lot# 01 328ES) buffered with citric-acid and Na₂HPO₄. 7H₂O was prepared immediately before use and added to each well. The optimal density was measured with Thermomax ELISA reader at 405 nm wavelength in kinetic mode for 10 minutes. Cytokine levels in the test sample were determined by comparison with reference standards.

### (iv) Anti-MUC1 antibody levels

Microtiter 96 well plates were coated with BP1-151-HSA conjugate (MUC1 24 amino acid peptide conjugated to HSA), or with Blend C (Blend C is a natural human MUC1 mucin purified from-ovarian cancer ascites). Serial dilutions of sera were incubated on the antigen coated plates at room temperature for 1 hr, after which the wells were -thoroughly-washed. Peroxidase-labeled goat anti-mouse IgG specific antibody was added and incubated at room temperature for 1 hr. Each plate was then washed and ABTS substrate was added. After 15 min the absorbance at 405 nm was measured on an ELISA reader.

### EXAMPLE 2

### MONO- AND DILIPIDATED MUC I ANTIGENIC PEPTIDES

The purpose of this example was to demonstrate that a liposomally-bound MUC I peptide with two lipid chains dramatically increases the production of anti-MUC I antibodies in immunized mice as compared to a MUC I peptide with one lipid.

MUC I peptides were chemically synthesized to contain one or two lipids. "BP1-217" has two liposerine residues attached at the carboxy terminus of the core peptidic sequence and "BP1-228" has only one liposerine attached to the carboxy terminus. The monolipopeptide and dilipopeptide were separately incorporated into liposomes and the resultant liposomal formulations were evaluated as vaccines.

### BP1-217: GVTSAPDTRPAPGSTAS(myristyl)S(myristyl)L

### BP1-228: GVTSAPDTRPAPGSTAS(myristyl)L

After at least two subcutaneous immunizations of C57BL/6 mice with the dilipopeptide liposomal vaccine, BP1-217, induced the T2, humoral response, producing very high levels of anti-MUC I immunoglobulin G (IgG). In contrast, a cellular response with very low levels of IgG produced was invoked in mice immunized two times with the monolipopeptide, BP1-228. For example, BP1-217 produced anti-MUC I IgG titers in the range of 1/72,000 to 1/218, 700 on BP1-151 HSA solid phase and 1/100-1/2700 titers on Blend C solid phase, whereas BP1-228 produced only low titers of IgG antibodies on BP1-151 HSA solid phase, and no antibodies were detected on Blend C solid phase. See Table 1, below.

BP1-228 or BLP-25, as monolipopeptides, produced very low or no antibody levels as compared to any MUC1 peptide with 2 lipid chains. All formulations tested are liposomal and contain lipid A adjuvant.

Anywhere from 40-100 µg of MUC1 lipopeptide can be used per immunization, although the present invention is not limited to these amounts, which may also vary according to the specific peptide used. 5 µg of MUC1 peptide into liposomes elicited a strong T1 immune response, but no antibody production. In clinical trials, a 1000 µg dose of BLP25 injected into patients was found to be very effective in eliciting a specific T cell proliferation.

It is also shown that quantitative rather than qualitative differences between lipid chains are important in eliciting T1 or T2 responses. That is, a humoral immune response can be elicited regardless of the type of lipid chain attached to the peptide. For instance, a MUC I peptide, "BP1-132," that has two palmitoyl lysine lipophilic amino acid residues attached to two adjacent lysine residues was also shown to induce humoral immunity. See Table 2.

### BP1-132: TAPPAHGVTSAPDTRPAPGSTAPPK(palmitate)K(palmitate)G

Mice: immunized twice with liposomal formulations containing BP1-132 invoked production of anti-MUC I antibodies with titers (IgG of 1/218,700 and tgM of 1/8100 to 1/72,900) similar to those recorded for BP1-217.

**Table 1: Protocol I346 IgG Antibody Titer Data Summary**

| Injected Material | Mouse # | BP1-151-HSA | | Blend C | |
|---|---|---|---|---|---|
| | | Log₂ Titer | Antibody Titer | Log₂ Titer | Antibody Titer |
| | | | | | |
| Group # 1 | 1 | 17.7 | 1/218,70 | 11.4 | 1/2700 |
| | 2 | 17.7 | O | < 6.6 | < 1/100 |
| **BLP16 Dilipo** | 3 | 16.2 | 1/218,70 | 9.8 | 1/900 |
| 400 µg/mL BP1-217 | 4 | 16.2 | O | 9.8 | 1/900 |
| 200 *µ*g/mL lipid A | 5 | 16.2 | 1/72,900 | 11.4 | 1/2700 |
| | | | 1/72,900 | | |
| | | | 1/72,900 | | |
| | | | | | |
| Group # 2 | 1 | 11.4 | 1/2700 | < 6.6 | < 1/100 |
| **BLP16 Manolipo** | 2 | 9.8 | 1/900 | < 6.6 | < 1/100 |
| 400 µg/mL BP1-228 | 3 | 9.8 | 1/900 | < 6.6 | < 1/100 |
| 200 *µ*g/mL lipid A | 4 | 9.8 | 1/900 | < 6.6 | < 1/100 |
| | 5 | < 6.6 | < 1/100 | < 6.6. | < 1/100 |
| | | | | | |
| | | | | | |
| Group # 3 | 1 | 6.6 | 1/100 | < 6.6 | <1/100 |
| | 2 | < 6.6 | <1/100 | < 6.6 | <1/100 |
| **BEP25** | 3 | 6.6 | 1/100 | < 6.6. | <1/100 |
| 400 µg/mL BP1-148 | 4 | < 6.6 | <1/100 | 8.2 | 1/300 |
| 200 *µ*g/mL lipid A | 5 | < 6.6 | <1/100 | < 6.6 | <1/100 |
| | | | | | |
| Group # 4 | 1 | < 6.6 | <1/100 | < 6.6 | <1/100 |
| | 2 | < 6.6 | <1/100 | < 6.6 | <1/100 |
| **Saline** | 3 | < 6.6 | <1/100 | < 6.6 | <1/100 |
| | 4 | 6.6 | 1/100 | < 6.6 | <1/100 |
| | 5 | < 6.6 | <1/100 | < 6.6 | <1/100 |

| | | | | | |
|---|---|---|---|---|---|
| C57BI/6 mice were immunized two times with liposomal formulation: BLP16 Dilipo containing MUC1 based lipopeptide (B1-217) and lipid A, or BLP1 6 Monolipo containing MUC1 based lipopeptide (BP1-228) and lipid A, or BLP25 containing MUC1-based lipopeptide (BP1-148) and lipid A BP1-217 GVTSAPDTRPAPGSTAS(Myristyl)S(Myristyl)L BP1-228 GVTSAPDTRPAPGSTAS(Myristyl)L BP1-148 STAPPAHGVTSAPDTRPAPGSTAPP-Lys(Palmitoyl)G | | | | | |

Two immunizations of dilipo-MUC I peptide liposomes stimulates mostly the T2, humoral, immune response, although there remains some cellular immune system activity, as seen by T cell proliferation and IFN-γ production. Nonetheless, the invention shows that liposomal administration of a MUC I peptide with two lipid chains (*e.g*., either palmitoyl lysine or myristyl serine lipid chains) attached provides a dramatic increase in antibody production and stimulation of the humoral immune system.

This accomplishment has not previously been-observed in any mammalian model

**Table 2: Protocol 1350B (2x immunization) IgM and IgG-Antibody Titer Data**

| | | **BPT-151 HSA** | | **Blend C** | |
|---|---|---|---|---|---|
| **Injected** | **Mouse** | | | | |
| **Material** | **#** | | | | |
| | | **IgM** | **IgG** | **IgM** | **IgG** |
| | | **Titer** | **Titer** | **Titer** | **Titer** |
| **BLP24Dilipo** | 1 | 1/72,900 | 1/218,700 | 1/100 | 1/300 |
| 400 *µ*g/mL BP1-132 | 2 | 1/24,300 | 1/218,700 | 1/100 | 1/300 |
| 200 *µ*g/mL lipid A | 3 | 1/8100 | 1/218,700 | 1/100 | 1/900 |
| X 2 immunization | 4 | 1/24,300 | 1/218,700 | 1/100 | 1/300 |
| | | | | | |
| **BLP25** | 1 | < 6.6 | < 1/100 | 1/100 | < 1/100 |
| 400 *µ*g/mL BP1- 148 | 2 | 6.6 | 1/100 | < 1/100 | < 1/100 |
| 200 *µ*g/mL lipid A | 3 | 8.2 | 1/300 | 1/100 | < 1/100 |
| X 2-immunization | 4 | < 6.6 < 6.6 | < 1/100 | 1/100 | < 1/100 |
| | | | | | |
| **Saline** | 1 | < 6.6 | < 1/100 | < 1/100 | < 1/100 |
| | 2 | < 6.6 | < 1/100 | < 1/100 | < 1/100 |
| X 2 immunization | 3 | < 6.6 | < 1/100 | < 1/100 | < 1/100 |
| | 4 | < 6.6 | < 1/100 | < 1/100 | < 1/100 |

| | | | | | |
|---|---|---|---|---|---|
| C57B1/6 mice were immunized two times with liposomal formulation: BLP24Dilipo, containing MUC1 based lipopeptide (BP1-132) and lipid A BLP25 containing MUC1 based lipopeptide (BP1-148) and lipid A BP1-182 TAPPAHGVTSAPDTRPAPGSTAPPK(Palmitoyl)K(Palmitoyl)L | | | | | |

To further characterize the observed phenomenon, MUC I transgenic mice were immunized with MUC I based liposomal formulations containing mono- or dilipopeptides. As shown in Table 3, a strong IgG antibody response was again observed upon immunization with the dilipopeptide (BP1-217), but only after four immunizations. This shows that C57B1/6 MUC I transgenic mice which are tolerogenic for MUC I antigen need more immunizations than normal C57B1/6 mice to break this tolerance and induce high levels of anti-MUC I IgG. Mice immunized with only the monolipopeptide (e.g., BP1-228), however, showed very low titers of IgG.

Thus, administration of a liposomal formulation comprising monolipo-MUC I peptide stimulates a cellular-response. Immunization with a dilipo-MUC I peptide liposomal formulation induces a humoral response. The creation of a liposome comprising monolipo-MUC I and dilipo-MUC I invokes both cellular and humoral immune responses upon immunization. Hence, modulation of the immune response can be achieved by selectively administering a particular liposomal formulation in a certain order.

**Table 3: Protocol I347C MUC1 Transgenic Mice-IgG Antibody Titer Data (4 Immunizations)**

| **Injected Material** | **Mouse #** | **BP1-151-HSA** | | **Blend C** | |
|---|---|---|---|---|---|
| | | **Log₂ Titer** | **Antibody Titer** | **Log₂ Titer** | **Antibody Titer** |
| Group # 1 | | | | | |
| **BLP16Dilipo** | 1 | 16.2 | 1/72,900 | < 6.6 | < 1/100 |
| 400 µg/mL BP1-217 | 2 | 16.2 | 1/72,900 | 6.6 | 1/100 |
| 200 *µ*g/mL lipid A | 3 | 14.6 | 1/24,300 | < 6.6 | < 1/100 |
| | 4 | 16.2 | 1/72,300 | < 6.6 | < 1/100 |
| | | | | | |
| Group # 2 | | | | | |
| **BLP16Monolipo** | 1 | 8.2 | 1/300 | 8.2 | 1/300 |
| 400 µg/mL BP1-228 | 2 | 9.8 | 1/900 | < 6.6 | < 1/100 |
| 200 *µ*g/mL lipid A | 3 | 9.8 | 1/900 | 8.2 | 1/300 |
| | 4 | < 6.6 | < 1/100 | < 6.6 | < 1/100 |
| Group # 3 | | | | | |
| | 1 | 11.4 | 1/2700 | < 6.6 | < 1/100 |
| **BLP25** | 2 | < 6.6 | < 1/100 | < 6.6 | < 1/100 |
| 400 µg/mL BP1-148 | 3 | 9.8 | 1/900 | < 6.6 | < 1/100 |
| 200 µg/mL lipid A | 4 | < 6.6 | < 1/100 | < 6.6 | < 1/100 |
| | | | | | |
| | | | | | |
| | 1 | < 6.6 | < 1/100 | < 6.6 | < 1/100 |
| Group # 4 | 2 | < 6.6 | < 1/100 | < 6.6 | < 1/100 |
| | 3 | < 6.6 | < 1/100 | < 6.6 | < 1/100 |
| Saline | 4 | < 6.6 | < 1/100 | < 6.6 | < 1/100 |
| | 5 | < 6.6 | < 1/100 | < 6.6 | < 1/100 |
| | | | | | |

| | | | | | |
|---|---|---|---|---|---|
| C57BI/6 MUC1 transgenic mice were immunized four times with liposomal formulation: BLP16 Dilipo containing MUC1 based lipopeptide (BP1-217) and lipid A or BLP16 Monolipo containing MUC1 based lipopeptide (BP1-228) and lipid A, or BLP25 containing MUC1 based-lipopeptide (BP1-148) and lipid A BP1-217 GVISAPDTRPAPGSTAS(Myristy)S(Myristyl)L BP1-228 GVTSAPDTRPAPGSTAS(Myristyl)L BP1-148 STAPPAHGVTSAPDTRPAPGSTAPP-Lys(Palmitoyl)G | | | | | |

### EXAMPLE 3

### MONO AND DILIPIDATED TUBERCULOSIS PEPTIDES

As was observed for MUC I monolipo and dilipopeptides, immunization with dilipo tuberculosis peptide dramatically increased antibody production. See Table 4.

**Table 4 Immune responses in C57BI/6 mice immunized two times with tuberculosis lipopeptide based liposomal vaccines**

| **Vaccine** | **T cell proliferation (CPM)** | **SI** | **IFN-g (pg/mL)** | **IgG TB dilipopep*** | **IgM TB dilipopep*** |
|---|---|---|---|---|---|
| TB Dilipo | 35064 | 13.2 | 9769 | 1/218,700 | 1/900 |
| TB Monolipo | 19501 | 16.2 | 2276 | 1/24,000 | <1/100 |
| Saline | 633 | 0.6 | 495 | <1/100 | <1/100 |

| | | | | | |
|---|---|---|---|---|---|
| *TB dilipopeptide DQVHFQPLPPAWKLSDALIK was used as a solid phase in ELISA assay | | | | | |

Two immunizations with dilipo-tuberculosis peptide increased the level of IgG titers dramatically as compared to titers that were induced after immunizations with monolipo-tuberculosis peptide. The results indicate that the presence of two lipids increases the titer that correlates to a humoral response by almost 10 times.

### EXAMPLE 4

### MONO- AND DILIPIDATED HEPATITIS B PEPTIDES

**Table-5: Protocol I368B Immune responses in mice immunized two times with hepatitis B mono- or dilipopeptide-liposomal vaccine**

| **Vaccine** | **T cell proliferati on (CPM)** | **IFN-g (pg/mL)** | **IgG Hepatitis B dilipopep*** | **IgM Hepatitis B dilipopep*** |
|---|---|---|---|---|
| Hepatitis B Dilipo | 3243 | 0 | 1/48,600 | 1/2700 |
| Hepatitis B Monolipo | 5242 | 1034 | 1/100 | <1/300 |
| Saline | 158 | 361 | <1/100 | <1/100 |

| | | | | |
|---|---|---|---|---|
| *Hepatitis B-dilipopeptide IRTPPAYRPPNAPILK(Palmitate)K(Palmitate)G was used as a solid phase in ELISA assay | | | | |

Two immunizations with dilipo- hepatitis B peptide increased dramatically the level of IgG titers, as compared to the titers that were induced after immunizations with monolipo- hepatitis B peptide. Similarly, the level of IgM antibodies is higher after dilipopeptide liposome formulation is used for immunization procedure.

### EXAMPLE 5

### MONO- AND DILIPIDATED MALARIA PEPTIDES

**Table 6: Protocol I368B Immune responses in mice immunized two times with malaria mono or dilipopeptide liposomal vaccine**

| **Vaccine** | **T cell proliferation (CPM)** | **IFN-g (pg/mL)** | **IgG Malaria dilipopep*** | **IgM Malaria dilipopep*** |
|---|---|---|---|---|
| Malaria Dilipo | 15075 | 1989 | 1/2700 | 1/2700 |
| Malaria Monolipo | 1280 | 1036 | 1/100 | <1/100 |
| Saline | 74 | 361 | <1/100 | <1/100 |

| | | | | |
|---|---|---|---|---|
| *Malaria dilipopeptide VTHESYQELVKKLEALEDAVK(Palmitate)K(Palmitate)G was used as a solid phase in ELISA assay | | | | |

Two immunizations with dilipo-malaria peptide increased the level of both IgG and IgM titers, as compared to the titers that were induced after immunizations with monolipo-malaria peptide. In this case malaria dilipopeptide vaccine induced stronger cell proliferation and IFN-γ levels as compared to monolipo- vaccine. This shows that the intensity of an immune response, *i*.*e.,* the cellular immune response, can be modulated by varying the number of lipids attached to the antigen.

### EXAMPLE 6

### VARYING THE RATIO OF MONO AND DILIPOPEPTIDES IN A LIPOSOME

By incorporating various ratios of mono and dilipopeptides into liposomes, it is possible to modulate the level and intensity of humoral responses. Table 7 summarizes IgM titers in C57BI/6 mice after two immunizations with various liposomal constructs. As expected, mice immunized with BLP25 monolipopeptide or saline (group 5 and 6) did not produce any detectable IgM titers. However, all liposomal formulations generated a comparable cellular immune response (data not shown).

The mixture of monolipo- and dilipopeptides at 3:1 ratio, respectively, did not significantly improve antibody titers (group 1); however, there is an increase in antibody titer, as compared to group 1 when mono- and dilipopeptides were incorporated into liposomes at 1:1 ratio. The most intense antibody response was observed in the mice immunized with a liposomal formulation containing MUC1 monolipo- and dilipopeptides at the ratio 1:3 (group 3).

Thus, by incorporating mono- and dilipopeptides at various ratios into liposomes it is possible to modulate the level of humoral responses.

**Table 7 : Protocol 1399B (2x immunization) IgM Antibody Titer Data**

| | | **IgM** | |
|---|---|---|---|
| **Injected Material** | **C57BI/6** | **BP1-151-H S A** | |
| X2 immunizations i.d. | **Mouse** # | **Log₂ Titer** | **Antibody Titer** |
| Gr.1 | | | |
| | 1 | 6.6 | 1/100 |
| **BLP25** | 2 | < 6.6 | < 1/100 |
| | 3 | 6.6 | 1/100 |
| Monolipo 300 µg/mL | 4 | 8.2 | 1/300 |
| Dilipo 100 µg/mL, 200µg/mL Lipid A | 5 | < 6.6 | < 1/100 |
| | | | |
| Gr.2 | | | |
| | 1 | 6.6 | 1/100 |
| **BLP25** | 2 | 6.6 | 1/100 |
| | 3 | 8.2 | 1/300 |
| Monolipo 200 µg/mL | 4 | 6.6 | 1/100 |
| Dilipo 200 µg/mL, 200µg/mL Lipid A | 5 | 8.2 | 1/300 |
| | | | |
| Gr.3 | | | |
| | 1 | 9.8 | 1/900 |
| **BLP25** | 2 | 14.6 | 1/24,300 |
| | 3 | 11.4 | 1/2700 |
| Monolipo 100 µg/mL | 4 | 9.8 | 1/900 |
| Dilipo 300 µg/mL, 200µg/mL Lipid A | 5 | 13 | 1/8100 |
| | | | |
| Gr.4 | | | |
| | 1 | 9.8 | 1/900 |
| **BLP25** | 2 | 9.8 | 1/900 |
| | 3 | 11.4 | 1/2700 |
| Dilipo 400 µg/mL | 4 | 11.4 | 1/2700 |
| 200µg/mL Lipid A | 5 | 9.8 | 1/900 |
| | | | |
| Gr.5 | | | |
| | 1 | < 6.6 | < 1/100 |
| **BLP25** | 2 | < 6.6 | < 1/100 |
| | 3 | < 6.6 | < 1/100 |
| Monolipo 400 µg/mL | 4 | 6.6 | 1/100 |
| 200µg/mL Lipid A | 5 | < 6.6 | < 1/100 |
| | | | |
| | | | |
| Gr.6 | 1 | < 6.6 | < 1/100 |
| | 2 | < 6.6 | < 1/100 |
| **Saline** | 3 | < 6.6 | < 1/100 |
| | 4 | < 6.6 | < 1/100 |
| | 5 | < 6.6 | < 1/100 |
| | | | |
| Monolipo [BP1-148 : STAPPAHGVTSAPDTRPAPGSTAPP-Lys(Pal)G] | | | |
| Dilipo [BP1-236 : STAPPAHGVTSAPDTRPAPGSTAPPK(Lipo)K(Lipo)G] | | | |

### EXAMPLE 7

### THE EFFECT OF LIPID CHAIN POSITION IN MUC1 LIPOPEPTIDE ON THE HUMORAL IMMUNE RESPONSE

The demonstration of a potent antibody response after immunization of mice with MUC1 lipopeptide with two liposerine residues attached to the carboxy terminus raises a question if this strong antibody response might be maintained, if the position or number of liposerine residues will be changed.

To answer this question several MUC1 lipopeptide constructs with different liposerine residue placements were synthesized. The data presented in Table 8 shows that only formulation #5, with three liposerine residues at carboxy terminus, was able to generate a potent anti-MUC1 IgG response. The insertion of a three-serine spacer between the two lipid chains (formulation #4) led to the decrease of the level of antibody responses.

Similarly, formulation #2 where only one lipid chain was inserted in the middle of the MUC1 peptide showed some low antibody titers, but those titers were much higher when two liposerine residues were inserted into-the central position of MUC1 molecule (formulation #1). Some low antibody titers were observed when the liposerine residues were placed in extreme positions: one on the carboxy terminus and one on the amino terminus (formulation #2).

The strongest antibody responses could be generated when two or three liposerine residues are placed at carboxy terminus. Changing the position of the liposerine residues still maintain some antibody titers, higher than BLP25 or saline (group 6 and 7) but significantly lower as compared to carboxy terminus location.

**Table 8 Protocol 1405 IgG Antibody titer data**

| **Injected Material** | **Mouse** # | **BP1-265** | |
|---|---|---|---|
| # immunizations | | **Log₂ Titer** | **Antibody Titer** |
| Gr.1 | | | |
| Formulation #1 (400 µg/mL BP1-271; 200 µg/mL lipid A) X 2 immunizations-i.d. | 1 | 11.40 | 1/2700 |
| | 2 | 11.40 | 1/2700 |
| | 3 | 9.80 | 1/900 |
| | 4 | 13.00 | 1/8100 |
| | 5 | 14.60 | 1/24,300 |
| | | | |

| Gr.2 | | | |
|---|---|---|---|
| Formulation #2 (400 µg/mL BP1-273; 200 µg/mL lipid A) X 2 immunizations - i.d. | 1 | <6.6 | <1/100 |
| | 2 | <6.6 | <1/100 |
| | 3 | 8.20 | 1/300 |
| | 4 | 8.20 | 1/300 |
| | 5 | 9.80 | 1/900 |
| | | | |

| Gr.3 | | | |
|---|---|---|---|
| Formulation #3 (400 µg/mL BP1-272; 200 µg/mL lipid A) X 2 immunizations - i.d. | 1 | 9.80 | 1/900 |
| | 2 | <6.6 | <1/100 |
| | 3 | 8.20 | 1/300 |
| | 4 | 8.20 | 1/300 |
| | 5 | <6.6 | <1/100 |
| | | | |

| Gr.4 | | | |
|---|---|---|---|
| Formulation #4 (400 µg/mL BP1-274; 200 µg/mL lipid A) X 2 immunizations-i.d. | 1 | 9.80 | 1/900 |
| | 2 | 8.20 | 1/300 |
| | 3 | 13.00 | 1/8100 |
| | 4 | 6.60 | 1/100 |
| | 5 | 11.40 | 1/2700 |
| | | | |

| Gr.5 | | | |
|---|---|---|---|
| Formulation #5 (400 µg/mL BP1-275; 200 µg/mL lipid A) X2 2 immunizations-i.d. | 1 | 14.60 | 1/24,300 |
| | 2 | 16.20 | 1/72,900 |
| | 3 | 16.20 | 1/72,900 |
| | 4 | 16.20 | 1/72,900 |
| | 5 | 16.20 | 1/72,900 |
| | | | |

| Gr.6 | | | |
|---|---|---|---|
| Formulation #6 (400 µg/mL BP1-148; 200 µg/mL lipid A) X 2 immunizations-i.d. | 1 | <6.6 | <1/100 |
| | 2 | <6.6 | <1/100 |
| | 3 | <6.6 | <1/100 |
| | 4 | 11.40 | 1/2700 |
| | 5 | <6.6 | <1/100 |
| | | | |

| Gr.7 | | | |
|---|---|---|---|
| Saline X 2 immunizations - i.d. | 1 | <6.6 | <1/100 |
| | 2 | <6.6 | <1/100 |
| | 3 | <6.6 | <1/100 |
| | 4 | <6.6 | <1/100 |
| | 5 | <6.6 | <1/100 |
| | | | |

| | | | |
|---|---|---|---|
| [BP1-265 : TSAPDTRPAPGSTAPPAHGVTSAPDTRPAPGSTAPPAHGVS(Lipo)S(Lipo)L] [BP1-271 : TSAPDTRPAPGSS(Lipo)S(Lipo)STSAPDTRPAPGS] [BP1-272 : TSAPDTRPAPGSS(Lipo)STSAPDTRPAPGS] [BP1-273 : S(Lipo)GVTSAPDTRPAPGSAS(Lipo)L] [BP1-274 : GVTSAPDTRPAPGSTAS(Lipo)SSSS(Lipo)L] [BP1-275 : GVTSAPDTRPAPGSTAS(Lipo)S(Lipo)S(Lipo)L] [BP1-148 : STAPDAHGVTSAPDTRPAPGSTAPP-Lys(Palmitoyl)]G | | | |

It is intended that the present invention covers the modifications and variations of this invention provided they come within the scope of the appended claims

## Claims

1. A liposome for use as a vaccine for treating cancer, tuberculosis, malaria or hepatitis B, which comprises an immunogenic dilipopeptide wherein a lipid is attached to each of two amino acids in the dilipopeptide.

2. The liposome for use according to claim 1, wherein a peptide portion of said dilipopeptide is derived from a cancer, tuberculosis, malaria or hepatitis B protein.

3. The liposome for use according to claim 2, wherein the peptide portion of said dilipopeptide is derived from a cancer protein.

4. The liposome for use according to claim 2 or claim 3, wherein the peptide portion comprises at least 5 contiguous amino acids comprising an epitope from an immunogenic portion of the protein.

5. The liposome for use according to any of claims 2 to 4, wherein the peptide portion of said dilipopeptide comprises a sequence, in whole or in part, selected from SEQ ID NOs. 1-4.

6. The liposome for use according to any preceding claim, which consists essentially of a dilipopeptide.

7. The liposome for use according to any of claims 3 to 6, wherein the peptide portion of said dilipopeptide is derived from MUC-1.

8. The liposome for use according to claim 7, wherein the peptide portion of said dilipopeptide comprises in whole or in part the sequence of SEQ ID NO. 1.

9. The liposome for use according to claim 7, wherein the peptide portion of said dilipopeptide comprises the sequence SGVTSAPDTRPAPGSTA or STAPPAHGVTSAPDTRPAPGSTAPP.

10. The liposome for use according to claim 7, wherein the lipids are positioned at either the N-terminus or the C-terminus of the MUC I peptide.

11. The liposome for use according to claim 7, wherein the dilipopeptide is GVTSAPDTRPAPGSTAS(myristyl)S(myristyl)L.

12. The liposome for use according to claim 7, wherein the dilipopeptide is TAPPAHGVTSAPDTRPAPGSTAPPK(Palmitoyl)K(Palmitoyl)L.

13. The liposome for use according to claim 7, wherein the dilipopeptide is TSAPDTRPAPGSTAPPAHGVTSAPDTRPAPGSTAPPAHGVS(Lipo)S(Lipo)L.

14. The liposome for use according to claim 7, wherein the dilipopeptide is TSAPDTRPAPGSS(Lipo)S(Lipo)STSAPDTRPAPGS.

15. The liposome for use according to claim7, wherein the dilipopeptide is S(Lipo)GVTSAPDTRPAPGSAS(Lipo)L.

16. The liposome for use according to claim 7, wherein the dilipopeptide is GVISAPDTRPAPGSTAS(Lipo)SSSS(Lipo)L.

17. The liposome for use according to claim 7, wherein the dilipopeptide is TAPPAHGVTSAPDTRPAPGSTAPPK(Palmitoyl)K(Palmitoyl)G.

18. The liposome for use according to any of claims 2 or 4 to 6, wherein the peptide portion of said dilipopeptide is derived from a tuberculosis protein.

19. The liposome for use according to claim 18, wherein the peptide portion of said dilipopeptide comprises the sequence DQVHFQPLPPAVVKLSDALIK.

20. The liposome for use according to any of claims 2 or 4 to 6, wherein the peptide portion of said dilipopeptide is derived from a hepatitis B protein.

21. The liposome for use according to claim 20, wherein the peptide portion of said dilipopeptide comprises the sequence IRTPPAYRPPNAPILK.

22. The liposome for use according to any of claims 2 or 4 to 6, wherein the peptide portion of said dilipopeptide is derived from a malaria protein.

23. The liposome for use according to claim 22, wherein the peptide portion of said dilipopeptide comprises the sequence VTHESYQELVKKLEALEDAVK.

24. The liposome for use according to any preceding claim, wherein each lipidated amino acid is a threonine, serine, lysine, arginine or cysteine.

25. The liposome for use according to any preceding claim, wherein each lipidated amino acid is independently lipidated with a myristyl, palmitoyl or lauryl moiety.

26. The liposome for use according to any preceding claim, wherein the lipids are positioned within three amino acids of the C-terminus of the dilipopeptide.

27. The liposome for use according to any preceding claim, for use in treating the individual by stimulating a humoral immune response to said dilipopeptide in the individual.

28. The liposome for use according to any preceding claim, for use in treating the individual by stimulating a cellular immune response to said dilipopeptidein the individual.

29. A liposomal composition for use as a vaccine for treating cancer, tuberculosis, malaria or hepatitis B, comprising at least one liposome as defined in any preceding claim, wherein the liposome further comprises a monolipopeptide.

30. The liposomal composition for use according to claim 29, wherein the percentage of said dilipopeptide varies from more than 0 to less than 100%, based on the total lipopeptides incorporated in said liposome.

31. The liposomal composition for use according to claim 29, wherein the percentage of said dilipopeptide incorporated in said liposome is 50%.

32. The liposomal composition for use according to claim 29, wherein the percentage of said dilipopeptide is from 50 to 99%.

33. The liposomal composition for use according to claim 29, wherein the monolipopeptide is STAPPAHGVTSAPDTRPAPGSTAPP-Lys(Palmitoyl)G.

34. The liposomal composition for use according to claim 29, wherein the monolipopeptide is GVTSAPDTRPAPGSTAS(Myristyl)L.

35. The liposomal composition for use according to claim 29, wherein the monolipopeptide is TSAPDTRPAPGSS(Lipo)STSAPDTRPAPGS.

36. The liposomal composition for use according to any of claims 29 to 35, wherein the monolipopeptide and dilipopeptide comprise different peptide epitopes.

37. The liposomal composition for use according to any of claims 29 to 35, wherein the monolipopeptide and dilipopeptide comprise the same peptide epitope.

38. The liposomal composition for use according to any of claims 29 to 37, further comprising an adjuvant.

39. A liposomal vaccine for use in stimulating an immune response in an individual which comprises an immunogenic dilipopeptide, said dilipopeptide comprising at least one peptide epitope, wherein a lipid is attached to each of two amino acids in the dilipopeptide.

## Patentansprüche

1. Ein Liposom zur Verwendung als ein Impfstoff zur Behandlung von Krebs, Tuberkulose, Malaria oder Hepatitis B, welches ein immunogenes Dilipopeptid umfasst, bei dem an jeder der zwei Aminosäuren in dem Dilipopeptid ein Lipid befestigt ist.

2. Das Liposom zur Verwendung gemäß Anspruch 1, wobei ein Peptidteil des Dilipopeptids von einem Krebs-, Tuberkulose-, Malaria- oder Hepatitis B-Protein abgeleitet ist.

3. Das Liposom zur Verwendung gemäß Anspruch 2, wobei der Peptidteil des Dilipopeptids von einem Krebsprotein abgeleitet ist.

4. Das Liposom zur Verwendung gemäß Anspruch 2 oder Anspruch 3, wobei der Peptidteil wenigstens 5 aufeinander folgende Aminosäuren umfasst, die ein Epitop aus einem immunogenen Teil des Proteins umfassen.

5. Das Liposom zur Verwendung gemäß irgendeinem der Ansprüche 2 bis 4, wobei der Peptidteil des Dilipopeptids eine vollständige oder einen Teil einer Sequenz umfasst, die ausgewählt ist aus den SEQ ID NO 1 - 4.

6. Das Liposom zur Verwendung gemäß irgendeinem vorhergehenden Anspruch, welches im Wesentlichen aus einem Dilipopeptid besteht.

7. Das Liposom zur Verwendung gemäß irgendeinem der Ansprüche 3 bis 6, wobei der Peptidteil des Dilipopeptids von MUC-1 abgeleitet ist.

8. Das Liposom zur Verwendung gemäß Anspruch 7, wobei der Peptidteil des Dilipopeptids die vollständige oder einen Teil der Sequenz von SEQ ID NO 1 umfasst.

9. Das Liposom zur Verwendung gemäß Anspruch 7, wobei der Peptidteil des Dilipopeptids die Sequenz SGVTSAPDTRPAPGSTA oder STAPPAHGVTSAPDTRPAPGSTAPP umfasst.

10. Das Liposom zur Verwendung gemäß Anspruch 7, wobei die Lipide entweder an dem N-Terminus oder dem C-Terminus des MUC-I-Peptids angeordnet sind.

11. Das Liposom zur Verwendung gemäß Anspruch 7, wobei das Dilipopeptid GVTSAPDTRPAPGSTAS(myristyl)S(myristyl)L ist.

12. Das Liposom zur Verwendung gemäß Anspruch 7, wobei das Dilipopeptid TAPPAHGVTSAPDTRPAPGSTAPPK(Palmitoyl)K(Palmitoyl)L ist.

13. Das Liposom zur Verwendung gemäß Anspruch 7, wobei das Dilipopeptid TSAPDTRPAPGSTAPPAHGVTSAPDTRPAPGSTAPPAHGVS(Lipo)S(Lipo)L ist.

14. Das Liposom zur Verwendung gemäß Anspruch 7, wobei das Dilipopeptid TSAPDTRPAPGSS(Lipo)S(Lipo)STSAPDTRPAPGS ist.

15. Das Liposom zur Verwendung gemäß Anspruch 7, wobei das Dilipopeptid S(Lipo)GVTSAPDTRPAPGSAS(Lipo)L ist.

16. Das Liposom zur Verwendung gemäß Anspruch 7, wobei das Dilipopeptid GVISAPDTRPAPGSTAS(Lipo)SSSS(Lipo)L ist.

17. Das Liposom zur Verwendung gemäß Anspruch 7, wobei das Dilipopeptid TAPPAHGVTSAPDTRPAPGSTAPPK(Palmitoyl)K(Palmitoyl)G ist.

18. Das Liposom zur Verwendung gemäß irgendeinem der Ansprüche 2 oder 4 bis 6, wobei der Peptidteil des Dilipopeptids von einem Tuberkuloseprotein abgeleitet ist.

19. Das Liposom zur Verwendung gemäß Anspruch 18, wobei der Peptidteil des Dilipopeptids die Sequenz DQVHFQPLPPAVVKLSDALIK umfasst.

20. Das Liposom zur Verwendung gemäß irgendeinem der Ansprüche 2 oder 4 bis 6, wobei der Peptidteil des Dilipopeptids von einem Hepatitis B-Protein abgeleitet ist.

21. Das Liposom zur Verwendung gemäß Anspruch 20, wobei der Peptidteil des Dilipopeptids die Sequenz IRTPPAYRPPNAPILK umfasst.

22. Das Liposom zur Verwendung gemäß irgendeinem der Ansprüche 2 oder 4 bis 6, wobei der Peptidteil des Dilipopeptids von einem Malariaprotein abgeleitet ist.

23. Das Liposom zur Verwendung gemäß Anspruch 22, wobei der Peptidteil des Dilipopeptids die Sequenz VTHESYQELVKKLEALEDAVK umfasst.

24. Das Liposom zur Verwendung gemäß irgendeinem vorhergehenden Anspruch, wobei jede lipidierte Aminosäure ein Threonin, Serin, Lysin, Arginin oder Cystein ist.

25. Das Liposom zur Verwendung gemäß irgendeinem vorhergehenden Anspruch, wobei jede lipidierte Aminosäure unabhängig voneinander mit einem Myristyl-, Palmitoyl- oder Laurylanteil lipidiert ist.

26. Das Liposom zur Verwendung gemäß irgendeinem vorhergehenden Anspruch, wobei die Lipide innerhalb von drei Aminosäuren des C-Terminus des Dilipopeptids angeordnet sind.

27. Das Liposom zur Verwendung gemäß irgendeinem vorhergehenden Anspruch zur Verwendung bei der Behandlung eines Individuums durch das Stimulieren einer humoralen Immunantwort auf das Dilipopeptid in dem Individuum.

28. Das Liposom zur Verwendung gemäß irgendeinem vorhergehenden Anspruch zur Verwendung bei der Behandlung eines Individuums durch das Stimulieren einer zellulären Immunantwort auf das Dilipopeptid in dem Individuum.

29. Eine liposomale Zusammensetzung zur Verwendung als ein Impfstoff zur Behandlung von Krebs, Tuberkulose, Malaria oder Hepatitis B, welche wenigstens ein Liposom wie in irgendeinem vorhergehenden Anspruch definiert umfasst, wobei das Liposom weiterhin ein Monolipopeptid umfasst.

30. Die liposomale Zusammensetzung zur Verwendung gemäß Anspruch 29, wobei der Prozentsatz des Dilipopeptids bezogen auf die gesamten Lipopeptide, die in dem Liposom enthalten sind, von mehr als 0 bis weniger als 100% variiert.

31. Die liposomale Zusammensetzung zur Verwendung gemäß Anspruch 29, wobei der Prozentsatz des Dilipopeptids, das in dem Liposom enthalten ist, 50% beträgt.

32. Die liposomale Zusammensetzung zur Verwendung gemäß Anspruch 29, wobei der Prozentsatz des Dilipopeptids von 50 bis 99% beträgt.

33. Die liposomale Zusammensetzung zur Verwendung gemäß Anspruch 29, wobei das Monolipopeptid STAPPAHGVTSAPDTRPAPGSTAPP-Lys(Palmitoyl)G ist.

34. Die liposomale Zusammensetzung zur Verwendung gemäß Anspruch 29, wobei das Monolipopeptid GVTSAPDTRPAPGSTAS(Myristyl)L ist.

35. Die liposomale Zusammensetzung zur Verwendung gemäß Anspruch 29, wobei das Monolipopeptid TSAPDTRPAPGSS(Lipo)STSAPDTRPAPGS ist.

36. Die liposomale Zusammensetzung zur Verwendung gemäß irgendeinem der Ansprüche 29 bis 35, wobei das Monolipopeptid und das Dilipopeptid verschiedene Peptidepitope umfassen.

37. Die liposomale Zusammensetzung zur Verwendung gemäß irgendeinem der Ansprüche 29 bis 35, wobei das Monolipopeptid und das Dilipopeptid dasselbe Peptidepitop umfassen.

38. Die liposomale Zusammensetzung zur Verwendung gemäß irgendeinem der Ansprüche 29 bis 37, welche weiterhin ein Adjuvans umfasst.

39. Ein liposomaler Impfstoff zur Verwendung beim Stimulieren einer Immunantwort in einem Individuum, welcher ein immunogenes Dilipopeptid umfasst, wobei das Dilipopeptid wenigstens ein Peptidepitop umfasst, wobei an jeder der zwei Aminosäuren in dem Dilipopeptid ein Lipid befestigt ist.

## Revendications

1. Liposome pour une utilisation en tant que vaccin pour traiter le cancer, la tuberculose, la malaria ou l'hépatite B, qui comprend un dilipopeptide immunogène dans lequel un lipide est attaché à chacun de deux acides aminés dans le dilipopeptide.

2. Liposome pour une utilisation selon la revendication 1, dans lequel une partie peptidique dudit dilipopeptide est dérivée d'une protéine du cancer, de la tuberculose, de la malaria ou de l'hépatite B.

3. Liposome pour une utilisation selon la revendication 2, dans lequel la partie peptidique dudit dilipopeptide est dérivée d'une protéine du cancer.

4. Liposome pour une utilisation selon la revendication 2 ou 3, dans lequel la partie peptidique comprend au moins 5 acides aminés contigus comprenant un épitope issu d'une partie immunogène de la protéine.

5. Liposome pour une utilisation selon l'une quelconque des revendications 2 à 4, dans lequel la partie peptidique dudit dilipopeptide comprend une séquence, en intégralité ou en partie, sélectionnée parmi SEQ ID NO: 1-4.

6. Liposome pour une utilisation selon l'une quelconque des revendications précédentes, qui consiste essentiellement en un dilipopeptide.

7. Liposome pour une utilisation selon l'une quelconque des revendications 3 à 6, dans lequel la partie peptidique dudit dilipopeptide est dérivée de MUC-1.

8. Liposome pour une utilisation selon la revendication 7, dans lequel la partie peptidique dudit dilipopeptide comprend, en intégralité ou en partie, la séquence de SEQ ID NO: 1.

9. Liposome pour une utilisation selon la revendication 7, dans lequel la partie peptidique dudit dilipopeptide comprend la séquence SGVTSAPDTRPAPGSTA ou STAPPAHGVTSAPDTRPAPGSTAPP.

10. Liposome pour une utilisation selon la revendication 7, dans lequel les lipides sont positionnés au niveau de l'extrémité N-terminale ou de l'extrémité C-terminale du peptide MUC I.

11. Liposome pour une utilisation selon la revendication 7, dans lequel le dilipopeptide est GVTSAPDTRPAPGSTAS(myristyle)S(myristyle)L.

12. Liposome pour une utilisation selon la revendication 7, dans lequel le dilipopeptide est TAPPAHGVTSAPDTRPAPGSTAPPK(Palmitoyle)K(Palmitoyle)L.

13. Liposome pour une utilisation selon la revendication 7, dans lequel le dilipopeptide est TSAPDTRPAPGSTAPPAHGVTSAPDTRPAPGSTAPPAHGVS(Lipo)S(Lipo )L.

14. Liposome pour une utilisation selon la revendication 7, dans lequel le dilipopeptide est TSAPDTRPAPGSS(Lipo)S(Lipo)STSAPDTRPAPGS.

15. Liposome pour une utilisation selon la revendication 7, dans lequel le dilipopeptide est S(Lipo)GVTSAPDTRPAPGSAS(Lipo)L.

16. Liposome pour une utilisation selon la revendication 7, dans lequel le dilipopeptide est GVISAPDTRPAPGSTAS(Lipo)SSSS(Lipo)L.

17. Liposome pour une utilisation selon la revendication 7, dans lequel le dilipopeptide est TAPPAHGVTSAPDTRPAPGSTAPPK(Palmitoyle)K(Palmitoyle)G.

18. Liposome pour une utilisation selon l'une quelconque des revendications 2 ou 4 à 6, dans lequel la partie peptidique dudit dilipopeptide est dérivée d'une protéine de la tuberculose.

19. Liposome pour une utilisation selon la revendication 18, dans lequel la partie peptidique dudit dilipopeptide comprend la séquence DQVHFQPLPPAVVKLSDALIK.

20. Liposome pour une utilisation selon l'une quelconque des revendications 2 ou 4 à 6, dans lequel la partie peptidique dudit dilipopeptide est dérivée d'une protéine de l'hépatite B.

21. Liposome pour une utilisation selon la revendication 20, dans lequel la partie peptidique dudit dilipopeptide comprend la séquence IRTPPAYRPPNAPILK.

22. Liposome pour une utilisation selon l'une quelconque des revendications 2 ou 4 à 6, dans lequel la partie peptidique dudit dilipopeptide est dérivée d'une protéine de la malaria.

23. Liposome pour une utilisation selon la revendication 22, dans lequel la partie peptidique dudit dilipopeptide comprend la séquence VTHESYQELVKKLEALEDAVK.

24. Liposome pour une utilisation selon l'une quelconque des revendications précédentes, dans lequel chaque acide aminé ayant subi une lipidation est une thréonine, une sérine, une lysine, une arginine ou une cystéine.

25. Liposome pour une utilisation selon l'une quelconque des revendications précédentes, dans lequel chaque acide aminé ayant subi une lipidation a indépendamment subi une lipidation avec un fragment myristyle, palmitoyle ou lauryle.

26. Liposome pour une utilisation selon l'une quelconque des revendications précédentes, dans lequel les lipides sont positionnés à moins de trois acides aminés de l'extrémité C-terminale du dilipopeptide.

27. Liposome pour une utilisation selon l'une quelconque des revendications précédentes, destiné à être utilisé pour traiter l'individu en stimulant une réponse immunitaire humorale contre ledit dilipopeptide chez l'individu.

28. Liposome pour une utilisation selon l'une quelconque des revendications précédentes, destiné à être utilisé pour traiter l'individu en stimulant une réponse immunitaire cellulaire contre ledit dilipopeptide chez l'individu.

29. Composition liposomale pour une utilisation en tant que vaccin pour traiter le cancer, la tuberculose, la malaria ou l'hépatite B, comprenant au moins un liposome tel que défmi dans l'une quelconque des revendications précédentes, le liposome comprenant en outre un monolipopeptide.

30. Composition liposomale pour une utilisation selon la revendication 29, dans laquelle le pourcentage dudit dilipopeptide varie de plus de 0 à moins de 100 %, sur la base des lipopeptides totaux incorporés dans ledit liposome.

31. Composition liposomale pour une utilisation selon la revendication 29, dans laquelle le pourcentage dudit dilipopeptide incorporé dans ledit liposome est de 50%.

32. Composition liposomale pour une utilisation selon la revendication 29, dans laquelle le pourcentage dudit dilipopeptide est compris entre 50 et 99 %.

33. Composition liposomale pour une utilisation selon la revendication 29, dans laquelle le monolipopeptide est STAPPAHGVTSAPDTRPAPGSTAPP-Lys(Palmitoyle)G.

34. Composition liposomale pour une utilisation selon la revendication 29, dans laquelle le monolipopeptide est GVTSAPDTRPAPGSTAS(Myristyle)L.

35. Composition liposomale pour une utilisation selon la revendication 29, dans laquelle le monolipopeptide est TSAPDTRPAPGSS(Lipo)STSAPDTRPAPGS.

36. Composition liposomale pour une utilisation selon l'une quelconque des revendications 29 à 35, dans laquelle le monolipopeptide et le dilipopeptide comprennent différents épitopes peptidiques.

37. Composition liposomale pour une utilisation selon l'une quelconque des revendications 29 à 35, dans laquelle le monolipopeptide et le dilipopeptide comprennent le même épitope peptidique.

38. Composition liposomale pour une utilisation selon l'une quelconque des revendications 29 à 37, comprenant en outre un adjuvant.

39. Vaccin liposomal pour une utilisation dans la stimulation d'une réponse immunitaire chez un individu, qui comprend un dilipopeptide immunogéne, ledit dilipopeptide comprenant au moins un épitope peptidique, dans lequel un lipide est attaché à chacun de deux acides aminés dans le dilipopeptide.
